(19) Europäisches Patentamt
European Patent Office
Office européen des brevets

(11) Veröffentlichungsnummer : **0 415 158 B1**

(12) **EUROPÄISCHE PATENTSCHRIFT**

(45) Veröffentlichungstag der Patentschrift :
**29.12.93 Patentblatt 93/52**

(51) Int. Cl.$^5$ : **C07C 209/36,** C07C 211/52

(21) Anmeldenummer : **90115434.4**

(22) Anmeldetag : **11.08.90**

(54) **Verfahren zur Herstellung von chlorsubstituierten aromatischen Aminen.**

(30) Priorität : **26.08.89 DE 3928329**

(43) Veröffentlichungstag der Anmeldung :
**06.03.91 Patentblatt 91/10**

(45) Bekanntmachung des Hinweises auf die Patenterteilung :
**29.12.93 Patentblatt 93/52**

(84) Benannte Vertragsstaaten :
**CH DE FR GB LI**

(56) Entgegenhaltungen :
**EP-A- 0 073 105**
**DE-A- 2 743 610**
**US-A- 3 145 231**
**US-A- 3 546 297**

(73) Patentinhaber : **BAYER AG**
**D-51368 Leverkusen (DE)**

(72) Erfinder : **Birkenstock, Udo, Dr.**
**Schneppersdelle 2**
**D-4030 Ratingen 8 (DE)**
Erfinder : **Kappler, Ulrich, Dr.**
**Rheindorfer Strasse 163c**
**D-4018 Langenfeld (DE)**
Erfinder : **Schmidt, Herbert**
**Im Hederichsfeld 52**
**D-5090 Leverkusen 3 (DE)**
Erfinder : **Zander, Jürgen, Dr.**
**Tannenweg 5**
**D-5090 Leverkusen 3 (DE)**

EP 0 415 158 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein verbessertes Verfahren zur Herstellung von chlorsubstituierten aromatischen Aminen.

Es ist bekannt, chlorsubstituierte aromatische Amine durch Hydrierung der entsprechenden Nitroverbindungen in Gegenwart von Edelmetallkatalysatoren herzustellen (siehe Ullmann, Band 7, 4. Aufl., Seite 570 (1973)). Es ist ferner bekannt, daß die bei dieser Hydrierung unerwünschten Dehydrohalogenierungen durch die Verwendung selektiv wirkender Edelmetall-Katalysatorsysteme, z.B. Platin und Ruthenium auf Trägermaterialien (siehe EP-OS 0 073 105 und US-PS 4 760 187) oder durch Zusätze von basischen Verbindungen, z.B. Ammoniak oder Morpholin (siehe DE-OS 2 743 610, US-PS 3 145 231, US-PS 3 361 819 und US-PS 3 291 832) oder durch partielle Katalysatorvergiftungen (siehe US-PS 4 059 627) weitgehend unterdrückt werden können.

Ferner ist bekannt, daß die Aktivität und Selektivität von Edelmetall-Katalysatoren durch Zusätze beeinflußt werden kann. So wird gemäß der US-PS 3 253 039 die Aktivität von Platin-Katalysatoren durch Zusatz von Silber verbessert, wogegen Chrom und Nickel auf den Katalysator vergiftend wirken.

Gemäß der US-PS 2 823 235 führen Nickel-Katalysatoren bei der Nitrobenzol-Hydrierung zur Bildung von Azo-, Azoxi- und Hydroazobenzolen und verstärken gemäß der US-PS 3 145 231 die Dehydrohalogenierung. In der US-PS 3 546 297 wird beschrieben, daß nur eine Kombination von $Cr^{3+}$- und $Ni^{2+}$-Verbindungen als Zusätze zu Platin-Katalysatoren eine Erhöhung der Katalysatoraktivität ohne gleichzeitige Erhöhung der Dehydrohalogenierungsrate bewirkt, während jeweils $Cr^{3+}$- oder $Ni^{2+}$-Verbindungen alleine uneffektiv sind. Gemäß der EP-OS 0 073 105 und der US-PS 4 760 187 wird bei der Verwendung von Platin-Katalysatoren, die Zusätze von $Cr^{3+}$- und $Ni^{2+}$-Verbindungen enthalten, bei der Hydrierung von 3,4-Dichlornitrobenzol die Bildung des unerwünschten Nebenproduktes 3,3',4,4' -Tetrachlorazobenzol (TCAB) deutlich gefördert.

Bei Verwendung von Dehydrohalogenierungs-Inhibitoren, z.B. Morpholin, muß gemäß US-PS 3 291 032 in der Regel ebenfalls ein erhöhter TCAB-Gehalt in durch Hydrierung von 3,4-Dichlornitrobenzol hergestelltem 3,4-Dichloranilin in Kauf genommen werden. Da TCAB und 3,3' ,4,4'-Tetrachlorazoxibenzol (TCAOB) bekannterweise dioxinähnliche Eigenschaften besitzen (Pergamon Series on Environmental Science, Vol. 5, S. 534-544; J. Soc. Occup. Med. (1981) 31, S. 158-163 und Science, Vol. 194, S. 627-630 (1976)), ist es erwünscht, die Bildung von TCAB und TCAOB soweit wie möglich zu vermeiden.

Es wurde nun ein Verfahren zur Herstellung von chlorierten aromatischen Aminen durch Umsetzung von chlorierten aromatischen Nitroverbindungen der Formel

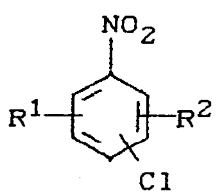

(I),

in der

$R^1$ und $R^2$ gleich oder verschieden sind und jeweils Wasserstoff, Methyl oder Chlor bedeuten,

mit Wasserstoff in Gegenwart eines Hydrierkatalysators bei 50 bis 250 bar, bei 120 bis 160°C und in Gegenwart eines aliphatischen Alkohols mit 1 bis 8 C-Atomen oder eines aromatischen Kohlenwasserstoffs mit 6 bis 10 C-Atomen und 0,2 bis 5 Gew.-% Ammoniak, bezogen auf die eingesetzte chlorierte aromatische Nitroverbindung, dadurch gekennzeichnet ist, daß man die Umsetzung in Gegenwart von Platin und Nickel und-oder Kobalt auf einem Aktivkohle-Träger durchführt.

Als Beispiele für chlorierte aromatische Nitroverbindungen der Formel (I) seien genannt: o-Nitrochlorbenzol, m-Nitrochlorbenzol, p-Nitrochlorbenzol, 2,4-Dichlornitrobenzol, 2,5-Dichlor-nitrobenzol, 3,4-Dichlor-nitrobenzol, 2,3,5-Trichlor-nitrobenzol, 2,4,6-Trichlor-nitrobenzol und 2-Chlor-4-nitrotoluol.

Es können auch beliebige Gemische von chlorierten aromatischen Nitroverbindungen der Formel (I) in das erfindungsgemäße Verfahren eingesetzt werden.

Vorzugsweise wird 3,4-Dichlornitrobenzol eingesetzt.

Das erfindungsgemäße Verfahren kann vorzugsweise bei 130 bis 150°C und vorzugsweise bei 80 bis 130 bar, durchgeführt werden.

Als Lösungsmittel kommen beispielsweise in Frage: Methanol, Ethanol, Propanol, Isopropanol, Butanol, Isobutanol, Pentanol, Isopentanol, Hexanol und Isohexanol (bevorzugt Isopropanol) und Benzol, Toluol und Xylol (bevorzugt Toluol).

Das Ammoniak kann im erfindungsgemäßen Verfahren gasförmig oder als Lösung eingesetzt werden. Als Ammoniaklösungen kommen z.B. wäßrige Lösungen oder Lösungen in einem der vorgenannten Losungsmittel in Frage.

Das Ammoniak kann bevorzugt in einer Menge von 0,3 bis 3 Gew.-%, besonders bevorzugt 0,7 bis 1,5 Gew,-%, bezogen auf die eingesetzte chlorierte aromatische Nitroverbindung, im Reaktionsgemisch vorliegen und ist bei kontinuierlicher Fahrweise gegebenenfalls entsprechend zu ergänzen.

Als Hydrierkatalysator wird in das erfindungsgemäße Verfahren ein Katalysator auf Aktivkohle als Trägermaterial eingesetzt. Der Katalysator enthält Platin, z.B. in einer Menge von 0,3 bis 7 Gew.-%, bevorzugt 0,5 bis 2 Gew.-%, und Nickel und/oder Kobalt, z.B. jeweils in Mengen von 1 bis 100 Gew.-%, bevorzugt 10 bis 30 Gew.-%, bezogen auf das Platin. Besonders bevorzugt sind Katalysatoren, die Platin und Nickel enthalten.

Der Aktivkohle-Träger für den Katalysator kann aus einem beliebigen porösen oder nichtporösen Material bestehen. Vorzugsweise werden handelsübliche, hochoberflächige und poröse Aktivkohlen pflanzlichen oder tierischen Ursprungs verwendet, wie sie z.B. von den Firmen Norit, Darco oder Degussa erhältlich sind.

Die Herstellung solcher Katalysatoren kann auf an sich bekannte Weise erfolgen, z.B. indem man einen Aktivkohleträger mit einer wäßrigen Platinsalzlösung, z.B. $H_2PtCl_6$-Lösung, imprägniert und gegebenenfalls mit einem Reduktionsmittel, z.B. Formaldehyd oder Hydrazin in alkalischer Lösung, zum Metall reduziert. Die Nickel- und/oder Kobalt-Verbindungen können in unterschiedlicher Weise auf den Aktivkohleträger aufgebracht werden. So kann der Platin enthaltende Aktivkohleträger mit einer wäßrigen Nickel- und/oder Kobalt-Salzlösung (z.B. Chloride, Nitrate oder Sulfate) versetzt werden, wobei unter alkalischen Bedingungen Nickel- und/oder Kobalt-Verbindungen praktisch quantitativ auf dem Aktivkohleträger abgeschieden werden. Alternativ können die Nickel- und/oder Kobalt-Verbindungen auch gemeinsam mit der Platin-Verbindung auf den Aktivkohle-Träger aufgebracht werden. Schließlich können auch geeignete Mischungen von auf Aktivkohle/Platin-, Aktivkohle/Nickel- und/oder Aktivkohle/Kobalt-Katalysatoren verwendet werden.

Das erfindungsgemäße Verfahren kann diskontinuierlich oder kontinuierlich betrieben werden.

Nach dem erfindungsgemäßen Verfahren werden chlorierte aromatische Aminoverbindungen der Formel (II) erhalten

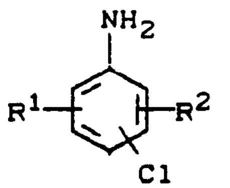

(II),

in der

$R^1$ und $R^2$ die bei Formel (I) angegebene Bedeutung haben.

Beispielsweise können so o-Chloranilin, m-Chloranilin, p-Chloranilin, 2,4-Dichloranilin, 2,5-Dichloranilin, 3,4-Dichloranilin, 2,3,5-Trichloranilin, 2,4,6-Trichloranilin und 2,4-Chlortoluidin hergestellt werden.

Solche chlorierten aromatischen Amine werden als Zwischenprodukte für die Herstellung von Pflanzenschutzmitteln und Farbstoffen benötigt.

Entscheidender Vorteil des erfindungsgemäßen Verfahrens ist, daß die Hydrierung sehr selektiv verläuft. Dehydrohalogenierungen finden nur in sehr geringem Umfang statt und gleichzeitig ist auch die Bildung von Nebenprodukten des Azobenzol-Typs deutlich geringer als bei Verfahren des Standes der Technik. Im Hinblick auf den eingangs geschilderten Stand der Technik ist dies besonders überraschend.

Beim erfindungsgemäßen Verfahren ist es besonders vorteilhaft, daß speziell bei der Hydrierung von 3,4-Dichlornitrobenzol die Bildung von TCAB als Nebenprodukt um ca. eine Zehnerpotenz niedriger ist als gegenüber dem in der DE-AS 2 743 610 beschriebenen Verfahren.

Somit kann auf eine aufwendige Nachreinigung erfindungsgemäß hergestellter chlorierter aromatischer Amine verzichtet werden, die aufgrund der gestiegenen Qualitätsanforderungen, insbesondere im Hinblick auf Gehalte an Nebenprodukten vom Azobenzol-Typ (z.B. TCAB), sonst notwendig gewesen wäre.

In den folgenden Beispielen sind Prozentangaben, somit nichts anderes vermerkt ist, Gewichtsprozente.

<u>Beispiele 1-11</u>

Die Ausprüfung der katalytischen Aktivität von verschiedenen Katalysatoren wurde nach einer standardisierten Methode der Hydrierung von 3,4-Dichlornitrobenzol in Gegenwart eines Isopropanol-Wasser-Gemisches und Ammoniak unter Druck in einem heizbaren Rührautoklaven aus Edelstahl (Werkstoff-Nr. 14580) durchgeführt.

Zur Austestung wurden 4 g Katalysator zur Hydrierung von 100 g 3,4-Dichlornitrobenzol in 300 g Isopropanol/Wasser/Gemisch, enthaltend 15 Gew.-% Wasser, und 10 ml wäßrige Ammoniaklösung, enthaltend 25 Gew.-% Ammoniak, eingesetzt.

Der Autoklav wurde mit Wasserstoff gespült und bei Raumtemperatur auf einen Wasserstoffdruck von 100 bar eingestellt.

Nach Prüfung auf Dichtigkeit wurden gleichzeitig der Rührer (450 U/min) und die Heizung eingeschaltet.

Die Absorption von Wasserstoff begann sofort unter schnellem Absinken des Wasserstoffdruckes und unter Anstieg der Reaktionstemperatur.

Nach jedem Abfallen des Wasserstoffdruckes auf 50 bar wurde wieder mit Wasserstoff auf 100 bar aufgedrückt. Die Absorption von Wasserstoff war nach einer Reaktionszeit von 5 bis 6 Minuten und einer Wasserstoffaufnahme von 160 bar beendet. Um eine vollständige Reduktion des 3,4-Dichlornitrobenzols sicherzustellen, wurde das auf 100°C erhitzte Reaktionsgemisch noch 20 Minuten bei 100°C und einem Wasserstoffdruck von 100 bar nachgerührt.

Danach wurde unter Rühren bis auf 30°C abgekühlt und die erhaltene Aminlösung vom Katalysator abfiltriert.

Der abfiltrierte Katalysator kann zurückgeführt und für weitere Hydrierungen von 3,4-Dichlornitrobenzol nach oben beschriebener Arbeitsweise wieder eingesetzt werden. In der folgenden Tabelle bedeutet "Rückführungen 0", daß frischer Katalysator eingesetzt wurde und "Rückführungen 1 bis 3", daß bereits ein einmal, zweimal bzw. dreimal in der gleichen Reaktion eingesetzter und rückgeführter Katalysator verwendet wurde. Die filtrierte Aminlösung wurde nach HPLC-Methoden hinsichtlich der Bildung von unerwünschten Nebenprodukten untersucht.

Entsprechend der oben beschriebenen Arbeitsweise wurde eine Serie von Reaktionen in Gegenwart verschiedener Hydrierkatalysatoren durchgeführt, die erfindungsgemäß Platin und Nickel und/oder Kobalt und zum Vergleich nur Platin, jeweils auf Aktivkohle-Träger enthielten.

Die unter gleichen Bedingungen und in der gleichen Apparatur erhältlichen Hydrierergebnisse sind reproduzierbar und können zur Beurteilung der katalytischen Aktivität und Selektivität der eingesetzten Katalysatoren untereinander verwendet werden.

Die bei der Laborausprüfung erhaltenen Ergebnisse sind in der nachfolgenden Tabelle zusammengefaßt:

Tabelle

| Beispiel | Katalystorzusammensetzung | Rückführungen | Nebenprodukte in der Aminlösung, die ca. 18-19 % DCA enthielt | | |
|---|---|---|---|---|---|
| | | | Chloranilin und Anilin [ppm] | TCAB [ppm] | TCAOB [ppm] |
| 1 | 1 % Pt + 0,1 % Ni/ Aktivkohle | 0 | 1054 | 8,6 | - |
| | | 1 | 1039 | 1,0 | - |
| | | 2 | 699 | 3,2 | - |
| | | 3 | 549 | 1,4 | - |
| 2 | 1 % Pt + 0,2 % Ni/ Aktivkohle | 0 | 1082 | 0,9 | - |
| | | 1 | 711 | 1,1 | - |
| | | 2 | 594 | 1,5 | - |
| | | 3 | 430 | 0,9 | - |
| 3 | 1 % Pt + 0,3 % Ni/ Aktivkohle | 0 | 1100 | - | - |
| | | 1 | 919 | 0,6 | - |
| | | 2 | 896 | 0,6 | - |
| | | 3 | 467 | 2,4 | - |
| 4 | 1 % Pt + 0,5 % Ni/ Aktivkohle | 0 | 2157 | - | - |
| | | 1 | 1553 | 0,7 | - |
| | | 2 | 1444 | 1,3 | - |
| 5 | 1 % Pt + 1,0 % Ni/ Aktivkohle | 0 | 2713 | - | - |
| | | 1 | 2509 | 0,5 | - |
| | | 2 | 2115 | 0,2 | - |
| | | 3 | 1266 | 0,3 | - |
| 6 | 1 % Pt + 0,2 % Co/ Aktivkohle | 0 | 689 | 41 | - |
| | | 1 | 461 | 5 | - |
| | | 2 | 449 | 0,6 | - |
| | | 3 | 297 | - | - |

Tabelle (Fortsetzung)

| Beispiel | | Katalysatorzusammensetzung | Rückführungen | Nebenprodukte in der Aminlösung, die ca. 18-19 % DCA enthielt: Chloranilin und Anilin [ppm] | TCAB [ppm] | TCAOB [ppm] |
|---|---|---|---|---|---|---|
| 7 | | 1 % Pt + 1 % Co/Aktivkohle | 0 | 562 | 5,9 | - |
| 8 | | 1 % Pt + 0,1 % Ni + 0,1 % Co/Aktivkohle | 0 | 1224 | 7,2 | - |
| | | | 1 | 1270 | 4,4 | - |
| 9 | | 1 % Pt + 0,2 % Ni + 0,2 % Co/Aktivkohle | 0 | 1372 | - | - |
| | | | 1 | 1176 | 0,8 | - |
| | | | 2 | 918 | 0,6 | - |
| 10 | | 1 % Pt + 0,5 % Ni + 0,5 % Co/Aktivkohle | 0 | 1203 | 0,4 | - |
| | | | 1 | 912 | 0,8 | - |
| | | | 2 | 599 | 0,8 | - |
| 11 | zum Vergleich | 1% Pt auf Aktivkohle | 0 | 3455 | 138 | 2,1 |
| | | | 1 | 1654 | 80 | 0,4 |
| | | | 2 | 827 | 81 | - |
| | | | 3 | 554 | 81 | - |

**Patentansprüche**

1. Verfahren zur Herstellung von chlorierten aromatischen Aminen durch Umsetzung von chlorierten aro-

matischen Nitroverbindungen der Formel

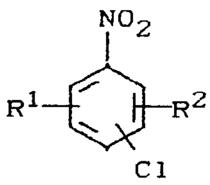

(I),

in der

$R^1$ und $R^2$ gleich oder verschieden sind und jeweils Wasserstoff, Methyl oder Chlor bedeuten,

mit Wasserstoff in Gegenwart eines Hydrierkatalysators bei 50 bis 250 bar, bei 120 bis 160°C und in Gegenwart eines aliphatischen Alkohols mit 1 bis 8 C-Atomen oder eines aromatischen Kohlenwasserstoffs mit 6 bis 10 C-Atomen und 0,2 bis 5 Gew.-% Ammoniak, bezogen auf die eingesetzte chlorierte aromatische Nitroverbindung, dadurch gekennzeichnet, daß man die Umsetzung in Gegenwart von Platin und Nickel und/oder Kobalt auf einem Aktivkohle-Träger durchführt.

2. Verfahren nach Anspruch 1, dadurch gekennzeichnet, daß man o-Nitrochlorbenzol, m-Nitrochlorbenzol, p-Nitrochlorbenzol, 2,4-Dichlor-nitrobenzol, 2,5-Dichlor-nitrobenzol, 3,4-Dichlor-nitrobenzol, 2,3,5-Trichlor-nitrobenzol, 2,4,6-Trichlor-nitrobenzol oder 2-Chlor-4-nitrotoluol einsetzt.

3. Verfahren nach Ansprüchen 1 und 2, dadurch gekennzeichnet, daß der Katalysator 0,3 bis 7 Gew.-% Platin und 1 bis 100 Gew.-% Nickel und/oder Kobalt, bezogen auf Platin, enthält.

4. Verfahren nach Ansprüchen 1 bis 3, dadurch gekennzeichnet, daß der Katalysator Platin und Nickel enthält.

5. Verfahren nach Ansprüchen 1 bis 4, dadurch gekenzeichnet, daß der Aktivkohleträger eine hochoberflächige, poröse Aktivkohle pflanzlichen oder tierischen Ursprungs ist.

## Claims

1. Process for the preparation of chlorinated aromatic amines by reaction of chlorinated aromatic nitro compounds of the formula

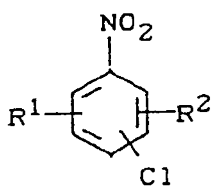

(I)

in which

$R^1$ and $R^2$ are identical or different and each denote hydrogen, methyl or chlorine,

with hydrogen in the presence of a hydrogenation catalyst under from 50 to 250 bar, at from 120 to 160°C and in the presence of an aliphatic alcohol having 1 to 8 carbon atoms or an aromatic hydrocarbon having 6 to 10 carbon atoms and 0.2 to 5 % by weight of ammonia, based on the chlorinated aromatic nitro compound employed, characterized in that the reaction is carried out in the presence of platinum and nickel and/or cobalt on an active charcoal support.

2. Process according to Claim 1, characterized in that o-nitrochlorobenzene, m-nitrochlorobenzene, p-nitrochlorobenzene, 2,4-dichloro-nitrobenzene, 2,5-dichloro-nitrobenzene, 3,4-dichloro-nitrobenzene, 2,3,5-trichloro-nitrobenzene, 2,4,6-trichloro-nitrobenzene or 2-chloro-4-nitrotoluene is employed.

3. Process according to Claims 1 and 2, characterized in that the catalyst contains 0.3 to 7% by weight of platinum and 1 to 100% by weight of nickel and/or cobalt, based on platinum.

4. Process according to Claims 1 to 3, characterized in that the catalyst contains platinum and nickel.

5. Process according to Claims 1 to 4, characterized in that the active charcoal support is a porous active charcoal of vegetable or animal origin having a high surface area.

## Revendications

1. Procédé de préparation d'amines aromatiques chlorées par réaction de dérivés aromatiques nitrés et chlorés de formule :

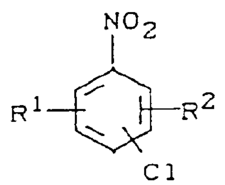

(I),

dans laquelle
$R^1$ et $R^2$, ayant des significations identiques ou différentes, représentent chacun l'hydrogène, un groupe méthyle ou le chlore,
avec l'hydrogène en présence d'un catalyseur d'hydrogénation à des pressions de 50 à 250 bar, et des températures de 120 à 160°C et en présence d'un alcool aliphatique en $C_1$-$C_8$ ou d'un hydrocarbure aromatique en $C_6$-$C_{10}$ et de 0,2 à 5% en poids d'ammoniac par rapport au dérivé aromatique nitré et chloré mis en oeuvre, caractérisé en ce que l'on effectue la réaction en présence de platine et de nickel et/ou de cobalt sur un support consistant en charbon actif.

2. Procédé selon la revendication 1, caractérisé en ce que l'on met en oeuvre l'o-nitrochlorobenzène, le m-nitrochlorobenzène, le p-nitrochlorobenzène, le 2,4-dichloronitrobenzène, le 2,5-dichloronitrobenzène, le 3,4-dichloronitrobenzène, le 2,3,5-trichloronitrobenzène, le 2,4,6-trichloronitrobenzène ou le 2-chloro-4-nitrotoluène.

3. Procédé selon les revendications 1 et 2, caractérisé en ce que le catalyseur contient de 0,3 à 7% en poids de platine et de 1 à 100% en poids de nickel et/ou de cobalt par rapport au platine.

4. Procédé selon les revendications 1 à 3, caractérisé en ce que le catalyseur contient du platine et du nickel.

5. Procédé selon les revendications 1 à 4, caractérisé en ce que le charbon actif servant de support est un charbon actif poreux à grande surface d'origine végétale ou animale.